# EUROPÄISCHE PATENTSCHRIFT

(11) **EP 1 622 862 B1**
(45) Veröffentlichungstag und Bekanntmachung des Hinweises auf die Patenterteilung: **13.07.2011**
(21) Anmeldenummer: 04727882.5
(22) Anmeldetag: 16.04.2004
(51) Int. Cl.: C07C 253/30, C07C 255/07

(54) **VERFAHREN ZUR ISOMERISIERUNG VON CIS-2-PENTENNITRIL ZU TRANS-3-PENTENNITRIL**
METHOD FOR THE ISOMERIZATION OF CIS-2-PENTENENITRILE TO FORM TRANS-3-PENTENENITRILE
PROCEDE D'ISOMERISATION DE CIS-2-PENTENENITRILE EN TRANS-3-PENTENENITRILE

(30) Priorität: 22.04.2003 DE 10323803
(43) Veröffentlichungstag der Anmeldung: 08.02.2006
(73) Patentinhaber: BASF SE, 67056 Ludwigshafen (DE)
(72) Erfinder: BARTSCH, Michael, 67433 Neustadt (DE); BAUMANN, Robert, 68161 Mannheim (DE); HADERLEIN, Gerd, 67269 Grünstadt (DE); FLORES, Miguel Angel, E-28300 Aranjuez (ES); JUNGKAMP, Tim, B-2950 Kapellen (BE); LUYKEN, Hermann, 67069 Ludwigshafen (DE); SCHEIDEL, Jens, 69493 Hirschberg (DE); SIEGEL, Wolfgang, 67117 Limburgerhof (DE); KUNSMANN-KEITEL, Dagmar, Pascale, 67117 Limburgerhof (DE)
(86) Internationale Anmeldenummer: PCT/EP2004/004040
(87) Internationale Veröffentlichungsnummer: WO 2004/094364

(56) Entgegenhaltungen:
- US-A- 3 526 654
- US-A- 3 852 325
- "Lehrbuch der anorganischen Chemie, 101 Seite 836" Holleman-Wiberg * Fussnote 5 *

## Beschreibung

Die vorliegende Erfindung betrifft ein Verfahren zur Isomerisierung von cis-2-Pentennitril zu trans-3-Pentennitril in Gegenwart von Aluminiumoxid als Katalysator, dadurch gekennzeichnet, dass das Aluminiumoxid eine BET-Oberfläche von mindestens 140 m²/g und höchstens 300 m²/g aufweist und man die Umsetzung bei einer Temperatur im Bereich von mindestens 120°C und höchstens 200°C in der Flüssigphase durchführt.

Bei der Hydrocyanierung von 3-Pentennitril zu Adipodinitril, das eine wichtige Ausgangsverbindung zur Herstellung von Polyamiden darstellt, in Gegenwart eines Ni(0) enthaltenden Katalysators entsteht bekanntermaßen als Nebenprodukt cis-2-Pentennitril. Dieses cis-2-Pentennitril kann üblicherweise - im Gegensatz zu 3-Pentennitril, wie trans-3-Pentennitril - in Gegenwart eines der genannten Ni(0) enthaltenden Katalysatoren nicht zu Adipodinitril hydrocyaniert werden und senkt somit die Ausbeute bei der Adipodnitril-Synthese.

Wünschenswert ist es demnach, das cis-2-Pentennitril in trans-3-Pentennitril zu isomerisieren, um dieses dann wieder in die Adipodinitril-Synthese zurückführen zu können.

US 3,526,654 offenbart die Isomerisierung von cis-2-Pentennitril in trans-3-Pentennitril in Gegenwart von Siliziumdioxid, Aluminiumoxid oder Natrium-Calcium-Silicat, wobei diese Katalysatoren in verschiedenen Modifikationen vorliegen können, in Flüssig- oder Gasphase bei Temperaturen im Bereich von 25°C bis 500°C. In Beispiel 3 wird die genannte Isomerisierung an Aluminiumoxid bei Raumtemperatur in der Flüssigphase beschrieben, wobei nach 6 Monaten ein Umsatz von 40 % beobachtet wurde. Diese Reaktionszeit ist für ein technisches Verfahren jedoch unwirtschaftlich.

Üblicherweise kann die Reaktionsgeschwindigkeit durch eine Anhebung der Reaktionstemperatur erhöht werden. Diese Maßnahme ist in der vorliegenden Isomerisierung von cis-2-Pentennitril in trans-3-Pentennitril nicht zweckdienlich, da bekanntermaßen im Falle von Pentennitrilen eine Erhöhung der Reaktionstemperatur innerhalb des in US 3,526,654 offenbarten Temperaturbereichs zur Bildung einer technisch inakzeptabel hohen Menge an Oligomeren und Polymeren führt.

Der vorliegenden Erfindung lag die Aufgabe zugrunde, ein Verfahren bereitzustellen, das die Isomerisierung von cis-2-Pentennitril zu trans-3-Pentennitril auf technisch einfache und wirtschaftliche Weise ermöglicht.

Demgemäß wurde das eingangs definierte Verfahren gefunden.

Das in das erfindungsgemäße Verfahren eingesetzte cis-2-Pentennitril kann nach an sich bekannten Verfahren erhalten werden, beispielsweise nach dem bereits eingangs beschriebenen Verfahren als Nebenprodukt bei der Hydrocyanierung von 3-Pentennitril, wie trans-3-Pentennitril oder cis-3-Pentennitril oder deren Gemische, oder ein solches 3-Pentennitril enthaltenden Mischungen, zu Adipodinitril.

In einer vorteilhaften Ausführungsform kann dabei das erfindungsgemäße Verfahren in ein solches Hydrocyanierungsverfahren zur Herstellung von Adipodinitril integriert werden.

In einer besonders vorteilhaften Ausführungsform kann eine solche Integration erfolgen, indem man
a) 3-Pentennitril oder eine Mischung, enthaltend 3-Pentennitril, in Gegenwart eines Ni(0) enthaltenden Katalysators nach an sich bekannten Verfahren zu Adipodinitril hydrocyaniert unter Erhalt von cis-2-Pentennitril als Nebenprodukt,
b) von der Produktmischung cis-2-Pentennitril abtrennt, beispielsweise durch Destillation,
c) cis-2-Pentennitril aus Schritt b) nach einem erfindungsgemäßen Verfahren isomerisiert unter Erhalt eines Produktstroms enthaltend trans-3-Pentennitril, daneben möglicherweise trans-2-Pentennitril oder cis-3-Pentennitril,
d) von dem in Schritt c) erhaltenen Produktstrom gegebenenfalls enthaltenes cis-2-Pentennitril abtrennt, beispielsweise durch Destillation, und in Schritt c) zurückführt unter Erhalt eines Reststroms,
e) den in Schritt d) erhaltenenen Reststrom in Schritt a) zurückführt.

Vorzugsweise kann man in Schritt a) als Ni(0) enthaltenen Katalysator einen solchen einsetzen, der neben Ni(0) weiterhin einen mehrbindigen Liganden, insbesondere einen Chelatliganden, der mehrere, wie zwei oder drei, zur Bindung an das besagte Ni(0) fähige dreibindige Phosphoratome, die unabhängig voneinander als Phosphin, Phosphinit, Phosphonit oder Phosphit vorliegen können, aufweist. Besonders vorteilhaft sollte der Katalysator weiterhin eine Lewissäure enthalten. Derartige Katalysatorsysteme sind an sich bekannt.

Erfindungsgemäß führt man die Isomerisierung in Gegenwart von Aluminiumoxid als Katalysator durch, wobei das Aluminiumoxid eine BET-Oberfläche von mindestens 100 m²/g aufweist.

Das Aluminiumoxid eine BET-Oberfläche von höchstens 300 m²/g aufweist.

Unter der BET-Obertläche wird im Sinne der vorliegenden Erfindung die spezifische Oberfläche bestimmt durch Messung der physisorbierten Gasmenge nach dem in: Brunauer, Emmett, Teller, J. Am. Chem. Soc. 60 (1938) Seite 309 beschriebenen Verfahren verstanden.

Das Alumniumoxid kann in reiner Form vorliegen.

Es ist möglich, Aluminiumoxid einzusetzen, das weitere Verbindungen enthält, wie Seltenerdenoxide, beispielsweise Ceroxid, Praeseodymoxid, Siliziumdioxid, Titandioxid, Eisenoxid, Alkalioxide, Erdalkalioxide oder deren Gemische. Solche Verbindungen können in Mengen von mindestens 10 Gew.-ppm bis höchstens 10 Gew.-%, bezogen auf die Summe aus Aluminiumoxid und solchen Verbindungen, enthalten sein.

Weiterhin können neben dem Oxid-Anion weitere Anionen, wie Hydroxid-Anionen, vorliegen.

Die Isomerisierung von cis-2-Pentennitril zu trans-3-Pentennitril kann vorteilhaft bis Teilumsatz durchgeführt werden unter Erhalt einer Mischung, die cis-2-Pentennitril und trans-3-Pentennitril enthält. Üblicherweise kann die Produktmischung weitere isomere Pentennitrile, wie trans-2-Pentennitril, cis-3-Pentennitril, 4-Pentennitril, 2-Methyl-2-Butennitril oder deren Gemische, enthalten.

Von einer solchen Mischung kann nicht umgesetztes cis-2-Pentennitril vorteilhaft abgetrennt werden, beispielsweise durch Destillation. Der an cis-2-Pentennitril abgereichte Reststrom kann vorzugsweise einer Hydrocyanierung zugeführt werden.

Es ist auch möglich, die bei der Isomerisierung erhaltene Produktmischung ohne Abreicherung von cis-2-Pentennitril einer Hydrocyanierung zugeführt werden.

Eine Isomerisierung in der Gasphase ist möglich; in einer vorteilhaften Ausführungsform kommt die Isomerisierung in der Flüssigphase in Betracht.

Die Temperatur bei der Isomerisierung beträgt mindestens 120°C.

Die Temperatur bei der Isomerisierung beträgt höchstens 200°C.

Die-Isomerisierung kann in Gegenwart eines flüssigen Verdünnungsmittels, insbesondere eines gegenüber den Pentennitrilen hinsichtlich der erfindungsgemäßen Isomerisierung inerten flüssigen Verdünnungsmittels, wie eines Kohelnwasserstoffs, durchgeführt werden. Bevorzugt kommt eine Isomerisierung in Abwesenheit eines solchen flüssigen Verdünnungsmittels in Betracht.

### Beispiele und Vergleichbeispiele 1-4.

cis-2-Pentennitril (Reinheit 98%) wurde mit 10 Gew.-%, bezogen auf cis-2-Pentennitril, Aluminiumoxid-Pulver versetzt und bei Normaldruck 7 Stunden unter Rückfluß erhitzt (126-144°C, Temperatur erhöhte sich im Verlaufe fortschreitenden Umsatzes).

Die Zusammensetzung wurde gaschromatographisch bestimmt. Die Ergebnisse sind in Tabelle 1 zusammengefaßt.

| Beispiel | BET-OF [m²/g] | c-2PN [Gew.-%] | t-2PN [Gew.-%] | t-3PN [Gew.-%] | c-3PN [Gew.-%] | Oligomere [Gew.-%] |
|---|---|---|---|---|---|---|
| Vgl.-Bsp.1 | 31,5 | 94,06 | 0,75 | 2,97 | 0,70 | 0 |
| Vgl.-Bsp2 | 72 | 70,25 | 15,07 | 10,96 | 2,17 | 0 |
| 2 | 106 | 57,24 | 19,95 | 17,89 | 3,23 | 0,17 |
| 3 | 250 | 56,04 | 19,18 | 19,27 | 3,12 | 0,84 |
| Vgf.-Bsp.4 | 349 | 39,3 | 34,1 | 18,5 | 5,1 | 1,4 |

**Tabelle 1**

| | |
|---|---|
| BET-OF: | BET-Oberfläche des jeweiligen Aluminiumoxids |
| c-2PN: | cis-2-Pentennitril |
| t-2-PN: | trans-2-Pentennitril |
| t-3-PN: | trans-3-Pentennitril |
| c-3-PN: | cis-3-Pentennitril |

| | |
|---|---|
| Die zu 100 % fehlenden Mengen sind Reste, z.B. isomere Nitrile. | |

Aus Vergleichsbeispiel 1 ist ersichtlich, dass mit einem Aluminiumoxid mit einer BET-Oberfläche von 31,5 [m²/g] keine technisch akzeptablen Isomerisierungsumsätze erzielt wurden.

## Patentansprüche

1. Verfahren zur Isomerisierung von eis-2-Pentennitril zu trans-3-Pentennitril in Gegenwart von Aluminiumoxid als Katalysator, **dadurch gekennzeichnet, dass** das Aluminiumoxid eine BET-Oberfläche von mindestens 100 m²/g und höchstens 300 m²/g aufweist und man die Umsetzung bei einer Temperatur im Bereich von mindestens 120°C und höchstens 200°C in der Flüssigphase durchführt.

## Claims

1. A process for isomerizing cis-2-pentenenitrile to trans-3-pentenenitrile in the presence of aluminum oxide as a catalyst, wherein the aluminum oxide has a BET surface area of at least 100 m²/g and at most 300 m²/g, and the reaction is carried out at a temperature in the range of from at least 120°C to at most 200°C in the liquid phase.

## Revendications

1. Procédé d'isomérisation de cis-2-pentène-nitrile en trans-3-pentène-nitrile en présence d'oxyde d'aluminium en tant que catalyseur, **caractérisé en ce que** l'oxyde d'aluminium présente une surface BET d'au moins 100 m²/g et d'au plus 300 m²/g et la réaction est réalisée à une température dans la plage allant d'au moins 120 °C à au plus 200 °C dans la phase liquide.
